# EUROPÄISCHE PATENTSCHRIFT

(11) **EP 2 848 580 B1**
(45) Veröffentlichungstag und Bekanntmachung des Hinweises auf die Patenterteilung: **24.08.2016**
(21) Anmeldenummer: 14184372.2
(22) Anmeldetag: 11.09.2014
(51) Int. Cl.: B08B 3/02, B65B 3/32, B67C 3/22, B67C 3/00, A61L 2/18, G01F 11/22

(54) **Vorrichtung zum Dosieren eines Füllprodukts in einen zu befüllenden Behälter**
Device for dosing a product into a container
Dispositif pour le dosage d'un produit dans un conteneur

(30) Priorität: 11.09.2013 DE 102013109968
(43) Veröffentlichungstag der Anmeldung: 18.03.2015
(73) Patentinhaber: Krones AG, 93073 Neutraubling (DE)
(72) Erfinder: Hausladen, Bastian, 93073 Neutraubling (DE); Goldbrunner, Christian, 93073 Neutraubling (DE); Engelsberger, Florian, 93073 Neutraubling (DE); Wuerzbauer, Georg, 93073 Neutraubling (DE); Faltermeier, Manfred, 93073 Neutraubling (DE); Koller, Stefan, 93073 Neutraubling (DE); Schubeck, Thomas, 93073 Neutraubling (DE)
(74) Vertreter: Nordmeyer, Philipp Werner

(56) Entgegenhaltungen:
- EP-A1- 1 707 530
- EP-A1- 2 567 902
- DE-B- 1 012 536
- DE-U1- 20 019 305
- DE-U1-202007 017 932
- US-A1- 2012 039 692

## Beschreibung

### Technisches Gebiet

Die vorliegende Erfindung betrifft eine Vorrichtung zum Dosieren eines Füllprodukts in einen zu befüllenden Behälter, bevorzugt eines viskosen oder pastösen Füllproduktes, wie beispielsweise Milch, Joghurt, Marmelade, Ketchup, Mayonnaise und/oder Babybrei mittels eines Rundlaufkolbendosierers.

### Stand der Technik

Rundlaufkolbendosierer sind im Stand der Technik bekannt, mittels welchen eine Dosierung viskoser oder pastöser Füllprodukte in zu befüllende Behälter durchgeführt werden kann. Hierzu ist es bekannt, in Rundlaufkolbendosierern mindestens eine Kolbendosiereinheit vorzusehen, welche üblicherweise einen Dosierzylinder und einen darin aufgenommenen Dosierkolben aufweist, mittels welchem das Füllprodukt aus einem Füllproduktreservoir in den Dosierzylinder eingesaugt wird und dann aus dem Dosierzylinder in den zu befüllenden Behälter ausgestoßen wird. Die Steuerung der Fluidströme wird dabei über eine Ventileinrichtung erreicht, welche zum Ansaugen des Füllprodukts aus dem Produktvorhaltebehälter den Produktvorhaltebehälter mit dem Dosierzylinder verbindet. In dem darauf folgenden Dosierschritt wird ein Auslassventil geöffnet, durch welches hindurch das Füllprodukt dann aus dem Dosierzylinder ausgetrieben wird. Beim Ansaugen des Füllprodukts in den Dosierzylinder ist das Auslassventil verschlossen und das Ansaugventil geöffnet. Beim Austreiben des Füllproduktes und damit beim Befüllen des zu befüllenden Behälters ist das Auslassventil geöffnet und das Ansaugventil geschlossen.

In einer alternativen Ausführungsform kann die Ventileinheit auch ein einziges Drehventil umfassen, mittels welchem die Verbindung des Dosierzylinders zwischen dem Produktvorhaltebehälter und dem Produktauslauf hin und her geschaltet werden kann.

Die Kolbendosiereinheit beziehungsweise eine Vielzahl an Kolbendosiereinheiten laufen bei einem Rundlaufkolbendosierer um eine zentrale Rotationsachse um, so dass eine entsprechende Vielzahl von zu befüllenden Behältern gleichzeitig befüllt werden kann. Bei einem Umlauf einer Kolbendosiereinheit um die Rotationsachse wird entsprechend der Ansaugvorgang und der Ausstoßvorgang durchgeführt, so dass nach der Durchführung einer Umdrehung des Rundläuferkolbenfüllers entsprechend ein zu befüllender Behälter mit dem vorgegebenen Füllproduktvolumen befüllt ist.

Bei der Abfüllung von Getränken und anderen Lebensmitteln ist die Hygiene der jeweiligen Rundlaufkolbenfüllervorrichtung von ausschlaggebender Bedeutung für die Qualität der abgefüllten Produkte. Entsprechend steht eine gute Reinigungsfähigkeit der Rundlaufkolbendosierer im Vordergrund.

Aus der EP 0 144 662 A1 ist eine Kolbendosiervorrichtung bekannt, bei welcher es möglich ist, einen oberen rotierenden Teil der Vorrichtung von einem unteren rotierenden Teil der Vorrichtung abzuheben, um dadurch eine Reinigung durchzuführen.

Die EP 1 707 530 A1 beschreibt einen versteiften Tisch für eine Behälterbehandlungsmaschine. Die DE 20 2007 017 932 U1 beschreibt eine Anordnung zum Bearbeiten von Getränkeeinheiten. Die US 2012/0039692 A1 beschreibt ein Schutzsystem für Behälterbehandlungsmaschinen. Die DE 200 19 305 U1 beschreibt eine Vorrichtung zur Außenreinigung von Behälterbehandlungsmaschinen.

### Darstellung der Erfindung

Ausgehend von dem bekannten Stand der Technik ist es eine Aufgabe der vorliegenden Erfindung, die Reinigungsfähigkeit einer Vorrichtung zum Dosieren eines Füllprodukts weiter zu verbessern.

Diese Aufgabe wird durch eine Vorrichtung zum Dosieren eines Füllprodukts in einen zu befüllenden Behälter mit den Merkmalen des Anspruchs 1 gelöst. Vorteilhafte Weiterbildungen ergeben sich aus den Unteransprüchen.

Entsprechend wird eine Vorrichtung zum Dosieren eines Füllprodukts in einen zu befüllenden Behälter, bevorzugt ein Rundlaufkolbendosierer mit mindestens einer Kolbendosiereinheit, umfassend einen Maschinengrundrahmen zum Halten mindestens einer Funktionseinheit vorgeschlagen. Erfindungsgemäß ist eine auf dem Maschinengrundrahmen aufliegende Wanne zur Aufnahme von Flüssigkeiten vorgesehen.

Dadurch, dass auf dem Maschinengrundrahmen eine Wanne zur Aufnahme und zum Ableiten von Flüssigkeiten aufliegt, kann erreicht werden, dass sämtliche Fluide und sämtliche Produktreste, welche beispielsweise von dem rotierenden Teil des Rundlaufkolbendosierers heruntertropfen, in der Wanne aufgefangen werden können, und entsprechend definiert abgeleitet werden können.

Entsprechend können Probleme dahingehend, dass Produktreste oder Reinigungsfluide über einen Maschinengrundrahmen auf den Boden abfließen, vermieden werden, denn durch das Bereitstellen der auf dem Maschinengrundrahmen aufliegenden Wanne kann entsprechend ein weitgehendes beziehungsweise vollständiges Aufnehmen und definiertes Ableiten der jeweiligen Flüssigkeiten und Produktresten erreicht werden.

Erfindungsgemäß weist der Maschinengrundrahmen mindestens eine gegenüber einer einen Wannenboden nachbildenden Ebene des Maschinengrundrahmens erhabene Aufnahme auf, an welcher eine Funktionseinheit anbringbar ist. Die erhabene Aufnahme ist bevorzugt auf einem Niveau oberhalb des Wannenbodens der Wanne angeordnet, welches mindestens auf dem maximalen Flüssigkeitsniveau beziehungsweise dem Überlaufniveau der Wanne liegt.

Unter Funktionseinheiten werden hier alle stationären, rotierenden und beweglichen Maschinenteile, Bauteile, Module und Einheiten verstanden, welche zum Betrieb der Vorrichtung, beispielsweise des Rundlaufkolbendosierers dienen. Beispielsweise wird darunter das eigentliche Karussell eines Rundlaufkolbendosierers, welches beispielsweise einen Produktvorhaltebehälter und an dessen Umfang angeordnete Kolbendosierer umfassen kann, verstanden. Stationäre Bauteile eines Rundlaufkolbendosierers wie beispielsweise eine Kurvenführung für die Zwangsführung der Dosierkolben mittels einer Kurvenrolle werden darunter ebenfalls verstanden. Weiterhin werden darunter auch Module zum Zuführen, Halten und Abführen der zu befüllenden Behälter beziehungsweise der befüllten Behälter verstanden. Weitere Zusatzmodule können beispielsweise zur Behälterbehandlung vorgesehen sein.

Die Wanne weist erfindungsgemäß eine Autnahmeabdeckung auf, welche die Aufnahme des Maschinengrundrahmens zumindest teilweise abdeckt. Die Wanne und insbesondere die Aufnahmeabdeckung folgt dann bevorzugt den Konturen der Aufnahme des Maschinengrundrahmens. Erfindungsgemäß dient mindestens eine Aufnahme des Maschinengrundrahmens als Auflager für die Wanne. Durch das Bereitstellen des Auflagers auf diese Weise kann entsprechend auf die Ausbildung weiterer Lager für die Wanne verzichtet werden.

Die Wanne ist bevorzugt zwischen der Aufnahme und einer an dieser angebrachten Funktionseinheit gehalten, bevorzugt durch Verschrauben eines Flansches der Funktionseinheit mit der Aufnahme. Durch die Verklemmung kann auf weitere Befestigungsmaßnahmen verzichtet werden.

Bevorzugt weist der Maschinengrundrahmen ein Metallgussmaterial, bevorzugt ein Metalldruckgussmaterial, auf. Auf diese Weise kann ein sehr stabiler Maschinengrundrahmen bereitgestellt werden, welcher es ermöglicht, die Massen, Gewichte und Vibrationen aufzunehmen und gleichzeitig auch ein gutes Widerlager für die gerade bei Kolbendosierern eingebrachten, sehr hohen Kräfte bereitzustellen. Der Maschinengrundrahmen ist dabei bevorzugt konstruktiv so ausgeführt, dass je nach Maschinenteilkreisdurchmesser unabhängig von der Rotationsrichtung und dem Zulauf und Ablauf der zu befüllenden Behälter eine Gussform zur Erstellung des Gussrahmens verwendet werden kann.

Der gesamte Maschinengrundrahmen wird bevorzugt von der Wanne überdeckt, um einen hygienisch einwandfreien Aufbau zu erreichen.

Der ein Metallgussmaterial aufweisenden Maschinengrundrahmen sorgt dabei für die nötige Stabilität und verhindert so ein Durchbiegen durch das Eigengewicht und durch die Lastaufnahme und Kraftableitung im Produktions- und Reinigungsbetrieb, insbesondere der Kolbendosiereinheiten. Entsprechend kann aufgrund der hohen Stabilität des ein Metallgussmaterial aufweisenden beziehungsweise aus Metallgussmaterial hergestellten Maschinengrundrahmens eine erhöhte Präzision beim Abfüllen erreicht werden, da Schwingungen reduziert beziehungsweise vermieden werden und die von den jeweiligen Kolbendosiereinheiten eingetragenen Kräfte zuverlässig abgestützt werden können.

Die auf dem Maschinengrundrahmen aufliegende Wanne umfasst bevorzugt Edelstahl und ist besonders bevorzugt vollständig aus Edelstahl gefertigt. Entsprechend ist die Wanne unempfindlich gegen Reinigungslaugen beziehungsweise Reinigungssäuren oder andere Reinigungsmittel und kann weiterhin hygienisch so abgereinigt werden, dass eine hygienische Befüllung der zu befüllenden Behälter ermöglicht wird.

Die auf dem Maschinengrundrahmen aufliegende Wanne weist besonders bevorzugt einen geneigten Boden auf, bevorzugt mit einer Neigung von 7% bis 10%, so dass die sich in der Wanne sammelnden Fluide, beispielsweise Reinigungsfluide oder Füllprodukt definiert ablaufen kann.

Der Maschinengrundrahmen weist in einer weiteren bevorzugten Ausgestaltung mindestens eine Aufnahme für mindestens ein Funktionselement der Vorrichtung, beispielsweise ein Funktionselement des rotierenden Teils eines Rundlaufkolbendosierers auf. Besonders bevorzugt sind mehrere solcher Aufnahmen zum Aufnehmen entsprechender Funktionseinheiten am Maschinengrundrahmen vorgesehen.

Um ein Beaufschlagen der Nahtstelle zwischen dem Maschinengrundrahmen beziehungsweise der Wanne und den jeweiligen Funktionseinheiten mit dem Reinigungsfluid oder anderen Fluiden zu vermeiden beziehungsweise deren schädliche Eigenschaften zu reduzieren, ist die Aufnahme gegenüber der Ebene, an welcher der Wannenboden anliegt, erhaben und die Wanne folgt der Form des Maschinengrundrahmens und insbesondere der Aufnahme.

Entsprechend ist die Aufnahme bevorzugt in Form eines Doms beziehungsweise eines zylindrischen Abschnittes, welcher sich nach oben erstreckt, in dem Maschinengrundrahmen vorgesehen. Die Aufnahme beziehungsweise die Aufnahmen weisen dabei eine Höhe über dem Boden der Wanne auf, welche dem maximal in der Wanne erreichbaren Flüssigkeitsniveau beziehungsweise dem Überlaufniveau der Wanne entspricht, beziehungsweise dieses übersteigt. Auf diese Weise kann sichergestellt werden, dass an der Schnittstelle zwischen der Funktionseinheit und der Aufnahme beziehungsweise dem erhabenen Bereich der Wanne, welcher über der erhabenen Aufnahme des Maschinengrundrahmens liegt, ein Benetzen des entsprechenden Schnittbereichs verringert beziehungsweise vermieden wird.

Die Aufnahme beziehungsweise die Aufnahmen dienen dabei als Auflager für die Wanne und die wird besonders bevorzugt zwischen der Aufnahme des Maschinengrundrahmens und der darauf montierten Funktionseinheit gehalten. Dies wird beispielsweise durch ein Verschrauben beispielsweise eines Flansches der Funktionseinheit erreicht, wobei die funktionale Einheit besonders bevorzugt über eine frontbündige Dichtung mit der Wanne in Verbindung tritt.

Entsprechend wird die Wanne quasi zwischen den domförmigen Aufnahmen und der Funktionseinheit verklemmt.

Die Wanne ist bevorzugt nahtlos geschweißt und gefalzt, und stellt entsprechend eine absolut dichte Wanne zur Aufnahme der jeweiligen Fluide bereit, welche besonders einfach und hygienisch einwandfrei gereinigt und sterilisiert werden kann.

Dadurch, dass die Wanne auf dem Rahmen aufliegt und mit diesem im Wesentlichen nur über die Aufnahmen verbunden ist, kommt es auch nicht zu Verspannungen beziehungsweise Verwerfungen der Wanne im Produktionsbetrieb beziehungsweise im Reinigungsbetrieb beziehungsweise wenn beispielsweise Reinigungsmedien mit hoher Temperatur aufgebracht werden.

Die Wanne dient weiterhin zur hermetischen Abdichtung des Maschinengrundrahmens beziehungsweise der unter der Wanne angeordneten Antriebe, Höhenverstellungs- oder Schmierungselemente und dem eigentlichen Produktions- beziehungsweise Dosierbereich. Durch die auf dem Maschinengrundrahmen aufliegende Wanne wird entsprechend eine hermetische und dichte Trennung vorgenommen, so dass eine hygienische Abfüllung erreicht wird.

Die Aufnahmen für die Antriebs- und Trägerelemente, beispielsweise für rotierende oder tragende Teile im Abfüllbereich, sind im Gussrahmen erhaben ausgeführt und dienen gleichzeitig als Auflager für die Wanne.

Auf der Wanne ist bevorzugt ein Maschinenschutz angeordnet, mittels welchem die Funktionseinheiten beziehungsweise Antriebs- und Trägerelemente für rotierende und tragende Teile eingehaust werden. Der Maschinenschutz ist dabei mit einem Labyrinthspritzschutz versehen, welcher einen zuverlässigen Spritzschutz und gleichzeitig einen einfachen Aufbau der Vorrichtung ermöglicht, ohne dass Elastomere zur Abdichtung eingesetzt werden müssen. Der Verzicht auf Elastomere bringt einen deutlich reduzierten Reinigungsaufwand und das bekannte Anbacken von Füllprodukt, beispielsweise von Marmelade oder von Babybrei an Elastomerdichtungen, kann auf diese Weise vermieden werden. Entsprechend kann eine bessere Abreinigbarkeit sowohl der Wanne als auch des Maschinenschutzes erreicht werden.

In einer besonders bevorzugten Weiterbildung ist eine Reinigungsvorrichtung vorgesehen, mittels welcher die einzelnen produktführenden Elemente sowie bevorzugt der durch den Maschinenschutz definierte Innenraum gespült werden kann. In einer besonders bevorzugten Ausprägung ein schwenkbarer Düsenstock vorgesehen, welcher zur Reinigung der Vorrichtung eingeschwenkt werden kann.

### Kurze Beschreibung der Figuren

Bevorzugte weitere Ausführungsformen und Aspekte der vorliegenden Erfindung werden durch die nachfolgende Beschreibung der Figuren näher erläutert. Dabei zeigen:
- Figur 1: eine schematische perspektivische Darstellung einer Vorrichtung mit einem Maschinengrundrahmen und einer darauf aufliegenden Wanne;
- Figur 2: eine Schnittansicht durch einen Bereich der Vorrichtung aus Figur 1;
- Figur 3: eine weitere Schnittansicht durch einen Bereich der Vorrichtung der Figuren 1 und 2;
- Figur 4: eine schematische perspektivische Darstellung einer Wanne, welche auf den Maschinengrundrahmen auflegbar ist;
- Figur 5: eine schematische perspektivische Ansicht der Vorrichtung mit angebrachten Funktionseinheiten in einer weiteren Ausführungsform;
- Figur 6: eine schematische Schnittansicht durch einen Randbereich der Wanne mit einem Maschinenschutz; und
- Figur 7: eine schematische Darstellung einer Verrohrung einer in der Vorrichtung anbringbaren Reinigungsanordnung.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen bezeichnet und auf eine wiederholte Beschreibung dieser Elemente wird in der nachfolgenden Beschreibung teilweise verzichtet, um Redundanzen zu vermeiden.

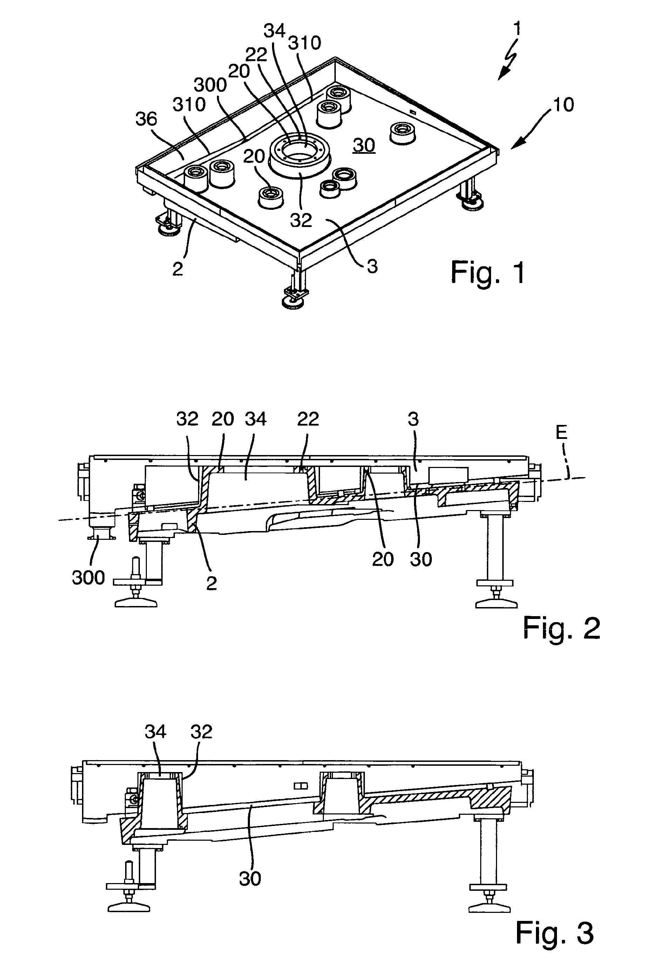

In den Figuren 1 bis 3 ist eine Vorrichtung 1 zum Dosieren eines Füllprodukts in einen zu befüllenden Behälter gezeigt, wobei in den Figuren lediglich die Unterkonstruktion 10 gezeigt ist, auf welcher Funktionseinheiten, besonders bevorzugt der stationäre und rotierende Teil eines Rundlaufkolbendosierers, aufgebaut werden können.

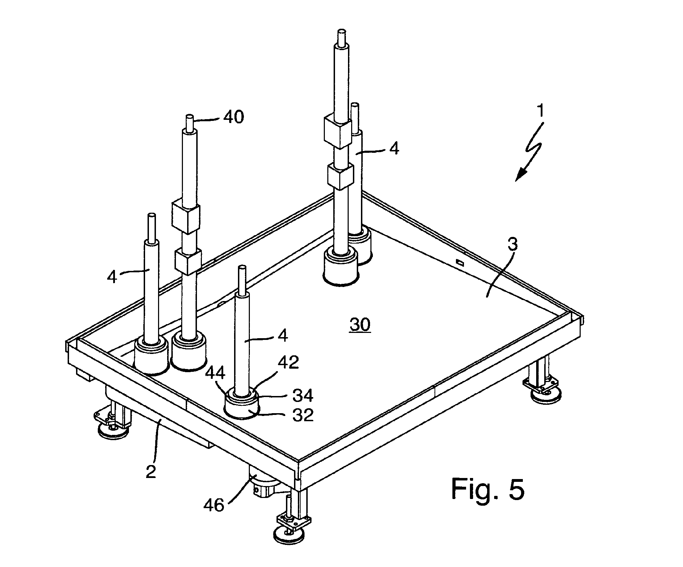

Die Vorrichtung 1 umfasst einen Maschinengrundrahmen 2, welcher zur Aufnahme der Funktionseinheiten der Vorrichtung 1 dient. Unter Funktionseinheiten werden hier alle stationären, rotierenden und beweglichen Maschinenteile, Bauteile, Module und Einheiten verstanden, welche zum Betrieb der Vorrichtung 1 dienen. Beispielsweise wird darunter das eigentliche Karussel eines Rundlaufkolbendosierers, welches beispielsweise einen Produktvorhaltebehälter und an dessen Umfang angeordnete Kolbendosierer umfassen kann, verstanden. Stationäre Bauteile eines Rundlaufkolbendosierers wie beispielsweise eine Kurvenführung für die Zwangsführung der Dosierkolben mittels einer Kurvenrolle werden darunter ebenfalls verstanden. Weiterhin werden darunter auch Module zum Zuführen, Halten und Abführen der zu befüllenden Behälter beziehungsweise der befüllten Behälter verstanden. Weitere Zusatzmodule können beispielsweise zur Behälterbehandlung vorgesehen sein. Ein schematischer Aufbau der Vorrichtung mit Funktionseinheiten 4 ist in der nachfolgend beschriebenen Figur 5 gezeigt.

Die Massen, Gewichte und Kräfte, welche von den Funktionseinheiten 4 ausgehen beziehungsweise welche abgestützt werden müssen, werden in den Maschinengrundrahmen 2 eingetragen und durch diesen aufgefangen und abgestützt. Beispielsweise werden die Kräfte, die durch die Rotation des rotierenden Maschinenteils eines mechanisch zwangsgeführten Rotationskolbenfüllers auf die stationäre Steuerkurve ausgeübt werden, zuverlässig in den Maschinengrundrahmen 2 eingeleitet und von diesem abgetragen.

Weiterhin ist eine Wanne 3 vorgesehen, welche zur Aufnahme und zum Ableiten von Flüssigkeiten vorgesehen ist. Mittels der Wanne 3 können im Dosierbetrieb der Vorrichtung 1 und insbesondere beim Betrieb eines Rotationskolbenfüllers heruntertropfende Füllproduktreste aufgenommen werden und abgeleitet werden, ohne dass diese auf einen umliegenden Hallenboden gelangen könnten. Hierdurch lässt sich eine besonders hygienische und sichere Abfüllung erreichen, da eventuell auslaufendes Füllprodukt die Umgebung nicht verschmutzt.

Weiterhin kann bei einem Reinigungsprozess, Sterilisierungsvorgang oder Sanitierungsprozess das anfallende Reinigungs-, Sterilisierungs- beziehungsweise Sanitierungsfluid von der Wanne 3 vollständig aufgefangen werden, so dass ein Austreten von Fluiden in die Umgebung nicht auftritt. Hierdurch lässt sich eine verbesserte und vereinfachte Reinigung der Vorrichtung 1 und bevorzugt eines Rundlaufkolbendosierers erreichen, derart, dass der Reinigungsprozess in einem abgeschlossenen Bereich stattfinden kann und die Umgebung damit nicht belastet wird. Die verwendeten Fluide können dabei besonders bevorzugt in einem Kreislaufprozess geführt und wiederverwendet werden, so dass die Umweltbelastung reduziert werden kann und Kosten gespart werden können.

Die Wanne 3 ist, wie sich beispielsweise aus den Schnittdarstellungen der Figuren 2 und 3 ergibt, auf den Maschinengrundrahmen 2 aufgelegt und bildet so eine hermetische Abdichtung des Dosierbereiches nach unten und insbesondere zum Hallenboden hin aus. Die Wanne 3 überspannt den gesamten Maschinengrundrahmen 2.

Die Wanne 3 weist einen Wannenboden 30 auf, welcher schräg gegenüber der Waagerechten angeordnet ist. Im gezeigten Ausführungsbeispiel liegt die Neigung bei ungefähr 7% bis 10%, so dass Reinigungsfluide und Füllproduktreste einfach vom Wannenboden 30 abfließen können und nicht in der Wanne 30 stehen bleiben.

Der Maschinengrundrahmen 2 definiert entsprechend eine schräg liegende Ebene E, auf welcher der schräge Boden 30 der Wanne 3 zumindest punktuell aufliegen kann. Die Wanne 3 ist dabei nicht vollflächig vom Maschinengrundrahmen 2 unterstützt, sondern liegt nur in einigen Positionen auf dem Maschinengrundrahmen 2 auf.

Der Maschinengrundrahmen 2 ist bevorzugt massiv und aus einem Metallgussmaterial ausgebildet, wodurch es ermöglicht wird, die hohen Kräfte, Massen und Vibrationen sowie das hohe Gewicht beispielsweise eines Rundlaufkolbendosierers aufzunehmen beziehungsweise abzutragen. Eine solche Aufnahme beziehungsweise Ableitung der jeweiligen Kräfte ist mit einem Metallgussrahmen beziehungsweise Druckgussrahmen besonders effizient möglich, insbesondere im Vergleich zu einer herkömmlichen Konstruktion mit Metallprofilen. Damit lässt sich insbesondere bei einem mechanisch zwangsgesteuerten Rundlaufkolbendosierer eine hohe Präzision der Abfüllung erreichen.

Der Maschinengrundrahmen 2 weist eine Mehrzahl von Aufnahmen 20 auf, welche die Funktionseinheiten des Rundlaufkolbendosierers aufnehmen können und über welche die jeweiligen Funktionseinheiten mit dem Maschinengrundrahmen 2 verbunden werden. So wird es möglich, sämtliche Funktionseinheiten 4, welche an den Aufnahmen 20 des Maschinengrundrahmens 2 gehalten werden, in durch den Maschinengrundrahmen 2 definierten Abständen zueinander zu halten und aufgrund der Ausbildung des Maschinengrundrahmens 2 aus Metallgussmaterial eine präzise und vibrationsarme Konstruktion zu schaffen.

Um ein einfaches Befestigen der Funktionseinheiten 4 an dem Maschinengrundrahmen 2 zu erreichen, sind an den Aufnahmen 20 Bohrungen 22 vorgesehen, welche zum zuverlässigen Verbinden der auf den Aufnahmen 20 angeordneten Funktionseinheiten dienen.

Wie aus den Figuren 1 bis 3 besonders gut zu erkennen, sind die Aufnahmen 20 erhaben gegenüber dem Wannenboden 30 sowie der den Wannenboden 30 nachbildenden Ebene E des Maschinengrundrahmens 2 ausgebildet. Entsprechend sind die Aufnahmen 20 als Dome ausgeformt, welche über den Wannenboden 30 herausstehen.

Es ist auf diese Weise möglich, die Anbindung einzelner Funktionseinheiten an die Aufnahmen 20 des Maschinengrundrahmens 2 in einem Bereich auszuführen, welcher oberhalb des Wannenbodens 30 liegt. Damit wird erreicht, dass bei einem Durchströmen des Wannenbodens 30, beispielsweise durch ein Reinigungsfluid beziehungsweise beim Vorliegen eines Fluids oder von Produktresten auf dem Wannenboden 30, die Schnittstelle zwischen der Anbindung 20 und der jeweiligen Funktionseinheit nicht unterhalb des dann in der Wanne 3 vorliegenden Flüssigkeitsspiegels liegt.

Die Höhe der Aufnahmen 20 beziehungsweise der durch die Aufnahmen 20 gebildeten Dome ist so gewählt, dass sie mindestens bei einer Überlaufhöhe oder einer maximal zulässigen Höhe des Fluidstandes in der Wanne 3 liegen.

Entsprechend sind, wie sich aus den Figuren 2 und 3 sofort ergibt, die Höhen der unterschiedlichen Aufnahmen 20 gegenüber dem Wannenboden 30 auch unterschiedlich hoch und nehmen entsprechend der Neigung des Wannenbodens 30 zu den tiefer gelegenen Bereichen des Wannenbodens 30 hin zu. Die einzelnen Aufnahmen 20 sind dabei durchgängig auf dem gleichen, waagerechten Niveau gehalten, um eine Montageebene für die Funktionseinheiten auszubilden. In einer bevorzugten Variante können sie aber auch unterschiedliche Höhen aufweisen, solange sie über den Wannenboden 30 herausstehen und damit ein den Wannenboden 30 entlang strömendes beziehungsweise auf diesem vorliegendes Fluid überragen.

Die Wanne 3 folgt in ihrer Kontur den Aufnahmen 20 derart, dass in der Wanne 3 Aufnahmenabdeckungen 32 vorgesehen sind, welche im Wesentlichen die Höhe der Aufnahmen 20 erreichen und welche die Aufnahmen 20 einkleiden. Die Aufnahmenabdeckungen 32 weisen eine Öffnung 34 auf, durch welche hindurch Bereiche der Aufnahme 20 zugänglich sind und durch welche hindurch sich beispielsweise eine Antriebswelle von einem unter der Wanne 3 angeordneten Antrieb zu einer über der Wanne 3 liegenden Funktionseinheit 4 erstreckt.

Da die Aufnahmeabdeckung 32 über einen Teil der Aufnahme 20 gezogen ist, liegt die Aufnahmeabdeckung 32 und damit ein Teil der Wanne 3 bei einer Montage der Funktionseinheit zwischen der Funktionseinheit und der Aufnahme 20 des Maschinengrundrahmens 2. Die Wanne 3 wird entsprechend über die Montage der Funktionseinheiten an der Aufnahme 20 dadurch gehalten, dass der die Aufnahme 20 überdeckende Teil der Aufnahmeabdeckung 32 zwischen der Funktionseinheit und der Aufnahme 20 verklemmt wird. In einer Variante, in der sich die Aufnahmeabdeckung 32 weiter in die Aufnahme 20 hereinzieht beziehungsweise diese vollständig überdeckt, kann auch eine Verbindung über Bohrungen in der Aufnahmeabdeckung 32, welche analog zu den Bohrungen 22 der Aufnahme 20 ausgeführt sind, und durch Verschrauben erreicht werden.

Die Wanne 3 liegt dabei auf dem Maschinengrundrahmen 2 auf und ist, außer im Bereich der Aufnahmen 20, nicht weiter mit dem Maschinengrundrahmen verschraubt oder befestigt.

Die Aufnahmen 20 des Maschinengrundrahmens 2 wirken entsprechend als Auflager für die Wanne 3. Die Wanne 3 ist damit zumindest bezüglich einer Verschiebung in der Ebene E im Wesentlichen mittels der Aufnahmenabdeckungen 32 formschlüssig an den Aufnahmen 20 gehalten.

Die Wanne 3 ist bevorzugt aus einem Edelstahlmaterial ausgebildet, derart, dass eine gute Korrosionsbeständigkeit und eine einfache Abreinigbarkeit der Wanne 3 gegeben ist.

Die Aufnahmenabdeckungen 32, der Wannenboden 30 und auch die Seitenwände 36 der Wanne 3 sind bevorzugt gefalzt und geschweißt und so ausgebildet, dass eine vollständig dichte Wanne 3 bereitgestellt wird, welche auf dem Wannenboden 30 von einem Fluid, beispielsweise einem überlaufenden Füllprodukt beziehungsweise Reinigungs- oder Sterilisierungsfluid durchflossen werden kann, ohne dass das Fluid nach außen austreten kann, oder dass es in einen Verbindungsbereich zwischen der Aufnahme 20 und den daran angeordneten Funktionseinheiten einfließen könnten. Die Aufnahmenabdeckungen 32 liegen damit sämtlich über einem Niveau oberhalb des Wannenbodens 30, welches mindestens der maximalen Füllhöhe der Wanne 3 entspricht.

Um einen zuverlässigen Ablauf der Fluide zu gewährleisten, weist die Wanne 3 einen Ablauf 300 auf, welcher am tiefsten Punkt der montierten Wanne 3 angeordnet ist. Um ein zuverlässiges Einlaufen des Fluids in den Ablauf 300 zu erreichen, sind zusätzlich Ablaufrinnen 310 vorgesehen, welche am tiefsten Ende des Wannenbodens 30 angeordnet sind, und welche zum Ablauf 300 hin so geneigt sind, dass sich auf dem Wannenboden 30 befindliche Fluide zuverlässig ablaufen.

Beispielsweise kann an der in den Figuren 1 bis 3 im zentralen mittleren Bereich angeordneten großen Aufnahme 20 das Hauptkarussell eines Rundläuferkolbendosierers angeordnet werden. An den kleineren Aufnahmen 20, welche die zentrale Aufnahme 20 umgeben, können entsprechend Zusatzaggregate oder andere Funktionseinheiten angeordnet werden.

Besonders bevorzugt ist es, einen Großteil der Antriebe, beispielsweise der Elektromotoren und/oder der pneumatischen Antriebe, unterhalb der Wanne 3 und entsprechend an der Unterseite des Maschinengrundrahmens 2 vorzusehen. Im Bereich der domförmigen Aufnahmen 20 ist hier entsprechend, wie sich beispielsweise auch aus den Schnittansichten der Figuren 2 und 3 ergibt, ausreichend Platz, um beispielsweise Elektromotoren und Getriebe aufnehmen zu können. Dies ermöglicht eine Anordnung der jeweiligen Antriebe in Bereichen, welche gegenüber Flüssigkeiten besonders gut geschützt sind, da über ihnen die hermetisch abgedichtete Wanne 3 angeordnet ist. Weiterhin sind die jeweiligen Antriebseinheiten unterhalb des Maschinengrundrahmens 2 beziehungsweise am Maschinengrundrahmen 2 gut zugänglich, da sie auf Bodenniveau angeordnet sind und eine Wartung beziehungsweise Reparatur durchgeführt werden kann, ohne dass der jeweilige Bediener Flüssigkeiten ausgesetzt wäre.

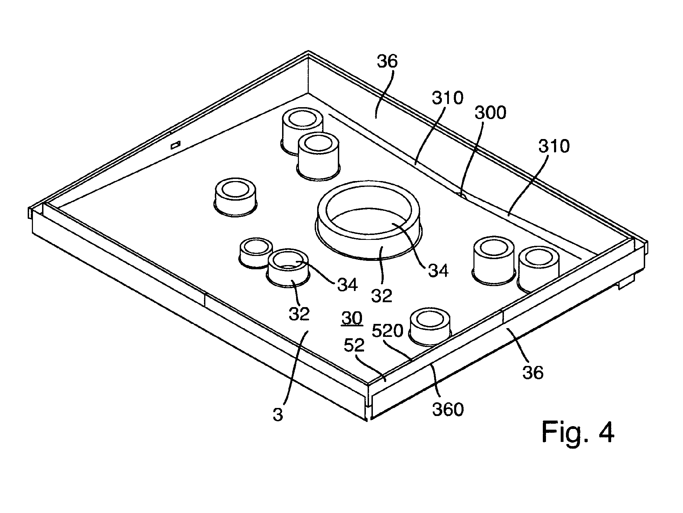

In Figur 4 ist schematisch die Wanne 3, welche in den Figuren 1 bis 3 in Verbindung mit dem Maschinengrundrahmen 2 gezeigt ist, allein gezeigt. Es ergibt sich, dass die Wanne 3 als hermetisch abgedichtete Edelstahlwanne ausgebildet ist, welche als Ganze entsprechend auf den Maschinengrundrahmen 2 aufgelegt wird, wobei die Aufnahmenabdeckungen 32 an den jeweiligen Aufnahmen 20 des Maschinengrundrahmens 2 aufliegen und die Aufnahmen 20 damit entsprechend als Auflager für die Wanne 3 dienen. Neben dem Einklemmen der Wanne 3 an den Aufnahmenabdeckungen 32 zwischen den Funktionseinheiten und den Aufnahmen 20 kommt keine weitere aktive Befestigung der Wanne 3 auf dem Maschinengrundrahmen 2 zum Zuge.

Gut zu erkennen in Figur 4 ist weiterhin der schräg ausgeführte Wannenboden 30, welcher dazu führt, dass die im niedrigen Teil der Wanne 3 angeordneten Aufnahmenabdeckungen 32 weniger hoch über den Wannenboden 30 überstehen, als die im tieferen Bereich der Wanne 3 angeordneten Aufnahmenabdeckungen 32.

In Figur 5 ist schematisch eine Vorrichtung 1 gezeigt, in welcher in einer weiteren Anordnung eine Wanne 3 auf einem Maschinengrundrahmen 2 aufgelegt ist. Funktionseinheiten 4, welche in Form von einen Abtrieb 40 aufweisenden Antrieben ausgeführt sind, sind auf den Aufnahmen 20 des Maschinengrundrahmens 2 befestigt, beispielsweise durch Aufflanschen und Festschrauben.

Es ist zu erkennen, dass ein Flansch 42 der Funktionseinheit 4 mit der Aufnahmenabdeckung 32 der Wanne 3 dichtend abschließt. Hierzu ist besonders bevorzugt auch eine nur schematisch angedeutete frontbündige Dichtung 44 am Flansch 42 der Funktionseinheit 4 vorgesehen. Entsprechend ergibt sich, dass aufgrund der Aufnahmenabdeckungen 32, welche gegenüber dem Wannenboden 30 erhaben ausgebildet sind, der Flansch 42 der Funktionseinheit 4 über dem maximal vorgesehenen Flüssigkeitsniveau in der Wanne 3 liegt. Dies gilt auch für die frontbündige Dichtung 44 zwischen der Funktionseinheit 4 und der Aufnahmenabdeckung 32.

Eventuell anfallendes Reinigungsfluid beziehungsweise Füllprodukt fließt lediglich an der Außenseite der Funktionseinheit 4, am Flansch 42 und an der frontbündigen Dichtung 44 auf die Aufnahmeabdeckung 32, kann aber keine Flüssigkeitssäule auf der frontbündigen Dichtung 44 ausbilden, welche einen besonderen Druck auf die Dichtung 44 ausüben würde. Auf diese Weise ist es möglich, dass eine besonders dichte und entsprechend eine besonders hygienische Ausführung erreicht werden kann.

Weiterhin ist die Anbindung der Funktionseinheiten 4 an dem Maschinengrundrahmen 2 zum einen mechanisch besonders stabil, zum anderen aber hygienisch einwandfrei durch die hermetische Abdichtung zwischen der Wanne 3 und der Funktionseinheit 4.

Dadurch, dass die Funktionseinheit 4 direkt an der Aufnahme 20 des Maschinengrundrahmens 2, welche domförmig ausgebildet ist, verbunden ist, kann eine mechanisch besonders stabile und schwingungsarme Ausführung erreicht werden. Damit ergibt sich auch, dass der Gesamtaufbau besonders stabil ist, da die hohen Gewichte, Massen und Kräfte, welche beim Abfüllen insbesondere mit einem mechanisch angesteuerten Rundlaufkolbendosierer auftreten, bei welchem der Dosierkolben in dem Dosierzylinder beispielsweise mittels einer Kurvenführung mechanisch zwangsgeführt wird, entsprechend gut über den Maschinengrundrahmen 2 abgetragen werden können.

Ein schematisch angedeuteter Antrieb 46 ist unterhalb der Wanne 3 am Maschinengrundrahmen 2 angeordnet, so dass er sicher unterhalb der hermetischen Abdichtung durch die Wanne 3 liegt und entsprechend nicht mit Fluiden beaufschlagt wird.

Mit anderen Worten ist zwischen dem Maschinengrundrahmen 2, der bevorzugt aus einem Metallguss beziehungsweise Druckguss besteht, um die hohen auftretenden Kräfte und Schwingungen zuverlässig aufnehmen zu können, und den jeweiligen Funktionseinheiten 4 eine hermetisch abdichtende Wanne 3 so angeordnet, dass das Austreten von Fluiden und Füllprodukt verhindert werden kann und gleichzeitig eine hygienische Abdichtung erreicht werden kann.

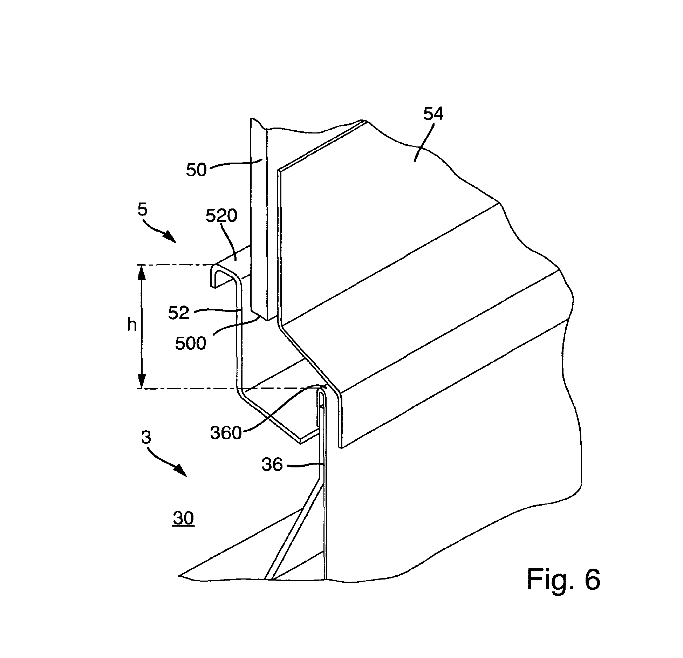

Um eine Abdichtung und einen Schutz auch der Funktionseinheiten beziehungsweise der oberhalb der Wanne 3 liegenden Maschinenteile zu erreichen, ist bevorzugt, wie beispielsweise in Figur 6 gezeigt, ein Maschinenschutz vorgesehen. Der Maschinenschutz ist dabei so auf der Seitenwand 36 der Wanne 3 aufgebaut, dass ein Labyrinthspritzschutz 5 ausgebildet wird. Der Labyrinthspritzschutz 5 umfasst zum einen den eigentlichen Maschinenschutzelement 50, welcher in Figur 6 schematisch als Glasscheibe gezeigt ist, welche aber durch jegliche andere plattenförmige Abschirmung, beispielsweise durch Edelstahlplatten, Plexiglas oder andere Wandelemente ausgebildet sein kann.

An der Seitenwand 36 der Wanne 3 ist entsprechend eine Rinne 52 angebracht, welche sich nach innen erstreckt, und welche mit ihrer Oberkante 520 um eine Höhe h über der Oberkante 360 der Seitenwand 36 liegt. Entsprechend ist die von der Rinne 52 ausgebildete Oberkante 520, welche innerhalb der Wanne 3 angeordnet ist, höher, als die Oberkante 360 der Außenwand 36 der Wanne 3.

Das Maschinenschutzelement 50 ist nun derart angeordnet, dass dessen Unterkante 500 unterhalb der Oberkante 520 der Rinne 52 angeordnet ist. Entsprechend ergibt sich hier bereits ein labyrinthförmiger Spritzschutz dahingehend, dass auf den Maschinenschutzelement 50 auftreffendes Fluid an dem Maschinenschutzelement 50 abfließt und danach in die Rinne 52 einfließt. Direkt im unteren Bereich des Maschinenschutzelementes 50 auftreffendes Fluid wird entweder von der Außenseite der Rinne 52 abgehalten, oder aber vom Maschinenschutzelement 50. Aufgrund der Tatsache, dass die Unterkante 500 des Maschinenschutzelementes 50 unterhalb der Oberkante 520 der Rinne 52 liegt, kann entsprechend ein Durchschlagen nach draußen von spritzendem Fluid nicht auftreten.

Eine weitere Verbesserung der Abdichtung kann dadurch erreicht werden, dass zusätzlich auf der Außenseite des Maschinenschutzelementes 50 ein Schutzprofil 54 aufgebracht wird, welches sowohl mit dem Maschinenschutzelement 50 als auch der Seitenwand 36 der Wanne 3 überlappt. Mittels dieses Schutzprofils 54 kann erreicht werden, dass auch aus der Rinne 52 kein Fluid auf die Außenseite herausspritzt.

Aufgrund der Tatsache, dass die Oberkante 360 der Außenwand 36 um die Höhe h niedriger ist, als die Oberkante 520 der Rinne 52, ist es in der gezeigten Ausführungsform auch möglich, das Maschinenschutzelement 50 als Tür auszubilden, welche dann nach außen öffnen kann.

Entsprechend wird ein Labyrinthspritzschutz 5 durch das entsprechende Hintereinanderschachteln unterschiedlich hoher Bauteile erreicht, so dass ein Austreten von Fluid beziehungsweise ein Herausspritzen von Fluid vermieden wird und gleichzeitig ein besonders einfacher Aufbau erreicht wird, welcher auch einfach abzureinigen ist.

Der in Figur 6 gezeigte Aufbau ermöglicht weiterhin, auf Elastomerdichtungen zwischen dem Maschinenschutzelement 50 und der Seitenwand 36 der Wanne 3 zu verzichten. Vielmehr lässt sich aufgrund der freien Zugänglichkeit auch der Unterkante 500 des Maschinenschutzelementes 50 eine effiziente und gezielte Reinigung erreichen, mittels welcher sämtliche Bereiche gut mit dem jeweiligen Reinigungs- beziehungsweise Sterilisierungsfluid gespült und beaufschlagt werden können. Der Verzicht auf eine Elastomerdichtung hat weiterhin den Vorteil, dass der hohe Verschleiß aufgrund der Anwendung von Reinigungsmedien unter hohen Temperaturen sowie das potentielle Anbacken von Füllproduktresten an den Elastomerdichtungen vermieden wird.

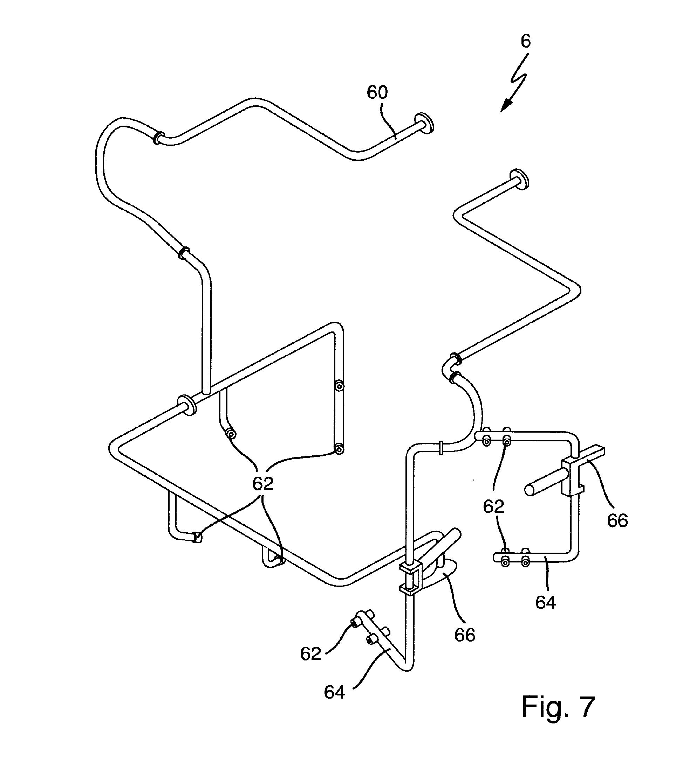

Figur 7 zeigt schematisch die Verrohrung für eine Reinigungsvorrichtung 6, welche in dem Rundlaufkolbendosierer der vorherigen Figuren untergebracht werden kann. Die Reinigungsvorrichtung 6 umfasst entsprechend eine Vielzahl von Transportrohren 60, welche in entsprechenden Reinigungsdüsen 62 enden, welche für die Reinigung und Sterilisierung der jeweiligen Rundlaufkolbendosiervorrichtung von Bedeutung sind. Über die Reinigungsdüsen 62 kann das Reinigungsfluid entsprechend an allen notwendigen Positionen aufgebracht werden und kann auch dazu dienen, die in Figur 6 beschriebenen Maschinenschutzelemente 50 mit dem Reinigungsfluid zu beaufschlagen.

Schwenkbare Reinigungsarme 64 sind vorgesehen, welche in bestimmten Maschinenbereichen zum Einbringen von Reinigungsfluid beziehungsweise Sterilisationsfluid vorgesehen sind. Ein solches Einschwenken eines schwenkbaren Reinigungsarms 64 ist dann von Bedeutung, wenn beispielsweise in einem Rotationskolbenfüller zwei drehbare Maschinenteile auseinandergefahren werden, um beispielsweise Ventile, Ventilkörper, Dosierkolben und Dosierzylinder vollständig reinigen zu können. Beim Auseinanderfahren der einzelnen Maschinenelemente kann dann der Reinigungsarm 64 in die entsprechende Reinigungsposition eingeklappt werden, und dann für eine vollständige Reinigung der entsprechenden Bereiche sorgen. Das Einschwenken der Schwenkarme 64 wird dabei bevorzugt über Schwenkvorrichtungen 66 erreicht, welche von der zentralen Anlagensteuerung angesteuert werden.

Aufgrund der Konstruktion mit dem aus einem Metallguss beziehungsweise Metalldruckguss bestehenden Maschinengrundrahmen 2 in Kombination mit der aus Edelstahl bestehenden Wanne 3 kann eine statisch einfache Wannenkonstruktion gewählt werden, da die Wanne selbst keinerlei Kräfte aufnehmen muss, sondern lediglich zu Abdichtungszwecken dient. Entsprechend kann mit relativ wenig Materialaufwand eine hermetisch abdichtende Edelstahlwanne aufgebaut werden, welche die Dichtungsfunktion und die hygienische Funktion vollständig ausfüllt.

Der Gussrahmen des Maschinengrundrahmens 2 ist so konzipiert, dass alle nötigen Aufnahmen 20, für die für den Betrieb der Vorrichtung 1 nötigen Funktionselemente 5, Baugruppen und Module vorgesehen sind, und entsprechend sämtliche Kräfte über den Maschinengrundrahmen 2 eingeleitet und abgetragen werden können. Insbesondere sind Aufnahmen 20 vorgesehen, an welchen das zentrale Karussell des Rundlaufkolbendosierers angeordnet ist, welches beispielsweise einen Produktvorhaltebehälter und an dessen Umfang angeordnete Kolbendosierelemente aufweist.

Weiterhin sind Aufnahmen 20 für den Transport und die Aufnahme der zu befüllenden Behälter vorgesehen, beispielsweise auch zum Einführen der zu füllenden Behälter und zum Ausführen der fertig befüllten Behälter.

Die entsprechenden Aufnahmen 20 sind erhaben im Maschinengrundrahmen 2 ausgebildet, derart, dass sie später auch über dem Wannenboden 30 der Wanne 3 erhaben ausgebildet sind. An der Unterseite des Maschinengrundrahmens 2 sind im Wesentlichen Antriebs-, Höhenverstellungs- und Schmierungselemente der Vorrichtung angebracht.

Die gefalzte und verschweißte Edelstahlwanne 3 wird auf den Maschinengrundrahmen 2 so aufgebracht, dass eine hermetische und flüssigkeitsdichte Trennung zwischen den am Maschinengrundrahmen 2 montierten Antriebselementen und dem dosiergutseitigen Dosierbereich erreicht wird. Die Wanne 3 wird dazu auf den Maschinengrundrahmen 2 aufgelegt, die Aufnahmen 20 für die Antriebs- und Trägerelemente im Dosierbereich sind im Maschinengrundrahmen erhaben ausgeführt und dienen als Auflager für die Wanne 3. Die Wanne 3 wird dann durch Flanschverschraubung der jeweiligen Funktionseinheiten an den Aufnahmen mit frontbündiger Abdichtung von unten fest mit dem Gussrahmen verbunden.

Die durch die Ausdomung erzielte Überhöhung der Auflageflächen an den Aufnahmen 20 beziehungsweise der Flansch- oder Abdichtungsstellen liegt über dem maximalen beziehungsweise dem Überlaufniveau der Wanne, wodurch die Dicht- beziehungsweise Flanschstellen nicht dauerhaft unter Flüssigkeit stehen.

An der Wanne können bei Bedarf Niveausensoren angebracht werden, mittels welchen das Flüssigkeitsniveau in der Ablaufrinne 310 beziehungsweise am Ablauf 300 oder aber auch dem Wannenboden 30 der Wanne 3 kontinuierlich gemessen werden kann. Bei Vorliegen eines zu hohen Pegels können entsprechende Maßnahmen ergriffen werden.

Die Rinnen 52, welche auf der Innenseite der Wanne 3 angebracht sind, sind selbstablaufend ausgebildet, so dass flüssige und fließfähige Medien, die sich in der umlaufenden Rinne 52 sammeln, auf den Wannenboden 30 ablaufen, und damit über den Auslauf 300 ausgetragen werden können.

Die Maschinenschutzelemente 50 oberhalb der Wanne 3 bestehen bevorzugt aus transparenten Materialien, zum Beispiel Einscheiben- oder Verbundsicherheitsglas, oder aus transparenten Kunststoffmaterialien, und teilweise auch aus Edelstahlblechsegmenten. Eingriffs- und Wartungszugänge zum Abfüllbereich der Vorrichtung 1 sind mittels schwenkbaren Türen begehbar ausgeführt. Die Maschinenschutzelemente 50 und die Schwenktüren sind so am Maschinengrundrahmen 2 befestigt, dass deren jeweiliger unterer Rand 500 niedriger liegt, als der innere, zur Tischwanne gerichtete Rand 520 der Rinne 52, so dass entsprechend eine labyrinthartige und spritzschützende Abdichtung erfolgt. Die Maschinenschutzelemente 50 und die Schwenktüren sind an am Maschinengrundrahmen 2 befestigten, eckständigen und/oder auf der Wanne 3 aufgesetzten Säulen befestigt, so dass die gesamte Maschine entsprechend spritzgeschützt ist.

Durch das Ausbilden des entsprechenden Maschinenschutzes kann weiterhin ein im Wesentlichen abgeschlossener Innenraum erreicht werden, welcher durch stetiges Spülen mit einem Spülgas beziehungsweise gefilterter Luft in einem definierten hygienischen Zustand gehalten werden kann, wobei die Luft dann über die zwischen den Maschinenschutzelementen 50 und der Wanne 3 angeordneten Spalte stetig und kontrolliert ausströmt. Durch das Bereitstellen der Wanne 3 kann mittels der Reinigungsvorrichtung 6 eine rezirkulierende Reinigung beziehungsweise rezirkulierende Sanitation durchgeführt werden, oder aber auch mit verlorenem Reinigungsmedium gearbeitet werden, wenn über den Ablauf 300 das ablaufende Reinigungsmedium verworfen wird.

Die Wanne 3 kann dabei auch als Puffer für das Reinigungs- oder Sanitationsmedium dienen, wobei dann über einen Niveausensor das maximale Füllniveau der Wanne 3 überwacht werden kann. Bei einer Reinigung im Rezirkulationsbetrieb, bei welcher die Wanne 3 als Puffer verwendet wird, ist es von besonderem Vorteil, dass die Verbindungsbereiche zwischen den jeweiligen Funktionseinheiten und dem Maschinengrundrahmen 2 oberhalb des Flüssigkeitsniveaus angeordnet sind, da dann insbesondere aggressive Reinigungsfluide nicht an einer Dichtung angreifen.

Soweit anwendbar, können alle einzelnen Merkmale, die in den einzelnen Ausführungsbeispielen dargestellt sind, miteinander kombiniert und/oder ausgetauscht werden, ohne den Bereich der Erfindung zu verlassen.

### Bezugszeichenliste

- 1: Vorrichtung zum Dosieren eines Füllprodukts in einen zu befüllenden Behälter
- 10: Unterkonstruktion
- 2: Maschinengrundrahmen
- 20: Aufnahme
- 22: Bohrung
- 3: Wanne
- 30: Wannenboden
- 32: Aufnahmeabdeckung
- 34: Öffnung
- 36: Seitenwand
- 300: Ablauf
- 310: Ablaufrinne
- 360: Oberkante Seitenwand
- 4: Funktionseinheit
- 40: Abtrieb
- 42: Flansch
- 44: frontbündige Dichtung
- 46: Antrieb
- 5: Labyrinthspritzschutz
- 50: Maschinenschutzelement
- 52: Rinne
- 54: Schutzprofil
- 500: Unterkante Maschinenschutzelement
- 520: Oberkante Rinne
- 6: Reinigungsvorrichtung
- 60: Transportrohr
- 62: Reinigungsdüse
- 64: schwenkbarer Reinigungsarm
- 66: Schwenkvorrichtung
- E: schräge Ebene des Maschinengrundrahmens
- h: Höhe

## Patentansprüche

1. Vorrichtung (1) zum Dosieren eines Füllprodukts in einen zu befüllenden Behälter, bevorzugt Rundlaufkolbendosierer mit mindestens einer Kolbendosiereinheit, umfassend einen Maschinengrundrahmen (2) zum Halten mindestens einer Funktionseinheit (4), wobei eine auf dem Maschinengrundrahmen (2) aufliegende Wanne (3) zur Aufnahme von Flüssigkeiten vorgesehen ist, und
der Maschinengrundrahmen (2) mindestens eine gegenüber einer einen Wannenboden (30) nachbildenden Ebene (E) des Maschinengrundrahmens (2) erhabene Aufnahme (20) aufweist, an welcher eine Funktionseinheit (4) anbringbar ist,
**dadurch gekennzeichnet, dass**
die Wanne (3) eine Aufnahmeabdeckung (32) aufweist, welche die Aufnahme (20) des Maschinengrundrahmens (2) zumindest teilweise abdeckt und die Aufnahme (20) des Maschinengrundrahmens (2) als Auflager für die Wanne (3) dient.

2. Vorrichtung (1) gemäß Anspruch 1, **dadurch gekennzeichnet, dass** die erhabene Aufnahme auf einem Niveau oberhalb des Wannenbodens (30) der Wanne (3) angeordnet ist, welches mindestens auf dem maximalen Flüssigkeitsniveau beziehungsweise dem Überlaufniveau der Wanne (3) liegt.

3. Vorrichtung (1) gemäß Anspruch 1 oder 2, **dadurch gekennzeichnet, dass** die Wanne (3) den Konturen der Aufnahme (20) des Maschinengrundrahmens (2) folgt.

4. Vorrichtung (1) gemäß einem der vorstehenden Ansprüche, **dadurch gekennzeichnet, dass** die Wanne (3) eine Aufnahmeabdeckung (32) aufweist, welche einen Teil der Aufnahme (20) überdeckt.

5. Vorrichtung (1) gemäß einem der vorstehenden Ansprüche, **dadurch gekennzeichnet, dass** der Maschinengrundrahmen (2) ein Metallgussmaterial aufweist und bevorzugt aus einem Metalldruckguss hergestellt ist.

6. Vorrichtung (1) gemäß einem der vorstehenden Ansprüche, **dadurch gekennzeichnet, dass** die Wanne (3) Edelstahl umfasst und bevorzugt aus Edelstahl besteht.

7. Vorrichtung (1) gemäß einem der vorstehenden Ansprüche, **dadurch gekennzeichnet, dass** die Wanne (3) zwischen der Aufnahme (20) und einer an dieser angebrachten Funktionseinheit (4) gehalten ist, bevorzugt durch Verschrauben eines Flansches (42) mit der Aufnahme (20).

8. Vorrichtung (1) gemäß einem der vorstehenden Ansprüche, **dadurch gekennzeichnet, dass** der Wannenboden (30) der Wanne (3) geneigt ist, bevorzugt mit einer Neigung von 7% bis 10% gegenüber der Waagerechten.

9. Vorrichtung (1) gemäß einem der vorstehenden Ansprüche, **dadurch gekennzeichnet, dass** auf der Wanne (3) ein Maschinenschutz (50) vorgesehen ist, welcher über einen Labyrinthspritzschutz (5) mit der Wanne (3) abgedichtet ist.

10. Vorrichtung (1) gemäß Anspruch 10, **dadurch gekennzeichnet, dass** der Labyrinthspritzschutz (5) eine Rinne (52) aufweist, welche eine Oberkante (520) aufweist, die auf einem höheren Niveau liegt, als die Unterkante (500) des Spritzschutzelementes (50).

11. Vorrichtung (1) gemäß einem der vorstehenden Ansprüche, **dadurch gekennzeichnet, dass** eine Reinigungsvorrichtung (6) vorgesehen ist, welche Reinigungsdüsen (62) aufweist und welche einen schwenkbaren Düsenstock (64) aufweist, welcher zur Reinigung eingeschwenkt werden kann.

## Claims

1. Device (1) for dispensing a filling product into a container to be filled, preferably a circulatory piston dispenser with at least one piston dispensing unit, comprising a basic machine frame (2) for holding at least one functional unit (4), wherein
a trough (3) sitting on the basic machine frame (2) is provided for collecting fluids, and the basic machine frame (2) has at least one attachment (20) which is raised with regard to a plane (E) of the basic machine frame (2) emulating a trough base (30) and on which a functional unit (4) can be applied,
**characterised in that**
the trough (3) has an attachment cover (32) which at least partially covers the attachment (20) of the basic machine frame (2) and the attachment (20) of the basic machine frame (2) acts as a support for the trough (3).

2. Device (1) according to claim 1, **characterised in that** the raised attachment is arranged at a level above the trough base (30) of the trough (3) which is at least at the maximum fluid level or the overflow level of the trough (3).

3. Device (1) according to claim 1 or 2, **characterised in that** the trough (3) follows the contours of the attachment (20) of the basic machine frame (2).

4. Device (1) according to any one of the above claims, **characterised in that** the trough (3) has an attachment cover (32) which covers a part of the attachment (20).

5. Device (1) according to any one of the preceding claims, **characterised in that** the basic machine frame (2) comprises cast metal material and is preferably produced from a metal die-casting.

6. Device (1) according to any one of the preceding claims, **characterised in that** the trough (3) contains stainless steel and preferably is produced from stainless steel.

7. Device (1) according to any one of the preceding claims, **characterised in that** the trough (3) is held between the attachment (20) and functional unit (4) applied thereon, preferably through screwing a flange (42) to the attachment (20).

8. Device (1) according to any one of the preceding claims, **characterised in that** the trough base (30) of the trough (3) is inclined, preferably with an inclination of 7% to 10% with regard to the horizontal.

9. Device (1) according to any one of the preceding claims, **characterised in that** on the trough (3), a machine protection (50) is provided which is sealed with the trough (3) via a labyrinth splash guard (5).

10. Device (1) according to claim 10 **characterised in that** the labyrinth splash guard (5) has a flume (52), which has an upper edge (520) lying at a higher level than the lower edge (500) of the splash guard element (50).

11. Device (1) according to any one of the preceding claims **characterised in that** a cleaning device (6) is provided which has cleaning nozzles (62) and which has a pivotable nozzle head (64) which can be pivoted in for cleaning.

## Revendications

1. Dispositif (1) pour doser un produit de remplissage dans un récipient à remplir, de préférence doseur à piston rotatif avec au moins une unité de dosage à piston, comprenant un cadre de base de machine (2) pour supporter au moins une unité fonctionnelle (4),
une cuve (3) qui repose sur le cadre de base de machine (2) étant prévue pour recevoir des liquides, et
le cadre de base de machine (2) comportant au moins un logement (20) qui est en relief par rapport à un plan (E), reproduisant un fond de cuve (30), du cadre de base de machine (2) et sur lequel une unité fonctionnelle (4) peut être agencée,
**caractérisé en ce que** la cuve (3) comporte un couvercle de logement (32) qui couvre au moins partiellement le logement (20) du cadre de base de machine (2) et **en ce que** le logement (20) du cadre de base de machine (2) sert de support pour la cuve (3).

2. Dispositif (1) selon la revendication 1, **caractérisé en ce que** le logement en relief est agencé à un certain niveau, au-dessus du fond de cuve (30) de la cuve (3), qui se trouve au moins au niveau de liquide maximal ou niveau de débordement de la cuve (3).

3. Dispositif (1) selon la revendication 1 ou 2, **caractérisé en ce que** la cuve (3) suit les contours du logement (20) du cadre de base de machine (2).

4. Dispositif (1) selon l'une des revendications précédentes, **caractérisé en ce que** la cuve (3) comporte un couvercle de logement (32) qui couvre une partie du logement (20).

5. Dispositif (1) selon l'une des revendications précédentes, **caractérisé en ce que** le cadre de base de machine (2) comporte une matériau coulé en métal et est fabriqué de préférence à partir d'une coulée en métal sous pression.

6. Dispositif (1) selon l'une des revendications précédentes, **caractérisé en ce que** la cuve (3) comprend de l'acier spécial et est de préférence constituée d'acier spécial.

7. Dispositif (1) selon l'une des revendications précédentes, **caractérisé en ce que** la cuve (3) est maintenue entre le logement (20) et une unité fonctionnelle (4) agencée sur celui-ci, de préférence par vissage d'une flasque (42) avec le logement (20).

8. Dispositif (1) selon l'une des revendications précédentes, **caractérisé en ce que** le fond de cuve (30) de la cuve (3) est incliné, de préférence avec une inclinaison de 7% à 10% par rapport à l'horizontale.

9. Dispositif (1) selon l'une des revendications précédentes, **caractérisé en ce qu'**il est prévu sur la cuve (3) une protection de machine (50) qui est étanchéifiée avec la cuve (3) par l'intermédiaire d'une protection anti-éclaboussure en labyrinthe (5).

10. Dispositif (1) selon la revendication 10, **caractérisé en ce que** la protection anti-éclaboussure en labyrinthe (5) comporte une goulotte (52) qui comporte une arête supérieure (520) qui se trouve à un niveau supérieur à celui de l'arête inférieure (500) de l'élément de protection anti-éclaboussure(50).

11. Dispositif (1) selon l'une des revendications précédentes, **caractérisé en ce qu'**il est prévu un dispositif de nettoyage (6) qui comporte des buses de nettoyage (62) et qui comporte un porte-buse pivotant (64) que l'on peut faire pivoter à l'intérieur pour le nettoyage.
